Europäisches Patentamt

European Patent Office

Office européen des brevets

(11) Publication number: **0 681 844 A1**

(12) **EUROPEAN PATENT APPLICATION published in accordance with Art. 158(3) EPC**

(21) Application number: 95901609.8

(22) Date of filing: 29.11.94

(86) International application number:
PCT/JP94/02003

(87) International publication number:
WO 95/15184 (08.06.95 95/24)

(51) Int. Cl.⁶: **A61K 51/02**, A61K 49/00

(30) Priority: 30.11.93 JP 300319/93

(43) Date of publication of application:
15.11.95 Bulletin 95/46

(84) Designated Contracting States:
BE DE ES FR GB IT

(71) Applicant: **KANEGAFUCHI KAGAKU KOGYO KABUSHIKI KAISHA**
2-4 Nakanoshima 3-chome
Kita-ku
Osaka-shi
Osaka 530 (JP)

(72) Inventor: **GEJYO, Fumitake**
38-1, Kobari 4-chome
Niigata-shi,
Niigata 950-21 (JP)
Inventor: **NAKATANI, Masaru**
Sansei-so,

31-17, Shioya-cho 6-chome,
Tarumi-ku
Kobe-shi,
Hyogo 655 (JP)
Inventor: **FURUYOSHI, Shigeo**
1-323-204, Kita-ochiai 1-chome,
Suma-ku
Kobe-shi,
Hyogo 654-01 (JP)
Inventor: **TAKATA, Satoshi**
1-3-704, Yoshida-cho 1-chome,
Hyogo-ku
Kobe-shi,
Hyogo 652 (JP)

(74) Representative: **Harding, Charles Thomas**
D. Young & Co.
21 New Fetter Lane
London EC4A 1DA (GB)

(54) SUBSTANCE FOR DETECTING AMYLOID DEPOSITION.

(57) A substance for detecting amyloid deposition and/or deposition site with excellent utility and safety, which is prepared by combining an amyloid-forming protein with a metal chelating substance. It is possible to obtain clear images of amyloid deposition and/or deposition site safely in a short time by labeling the invention substance with a radio-isotope via the metal chelating substance.

Rank Xerox (UK) Business Services
(3.10/3.09/3.3.4)

R-shoulder ↘

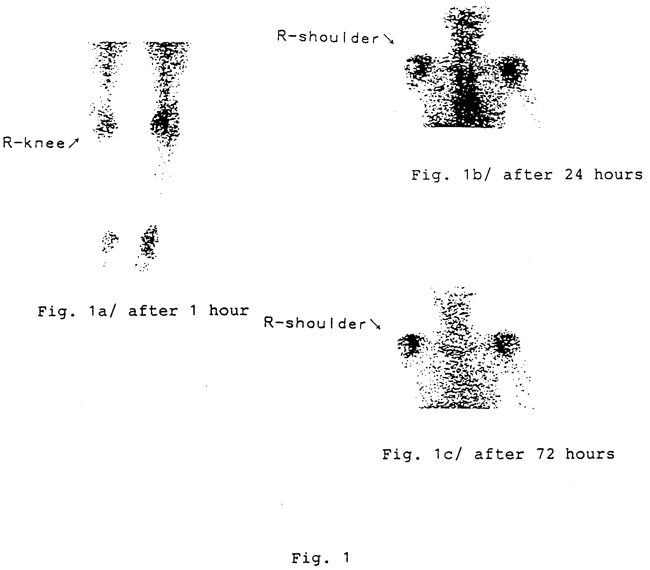

Fig. 1b/ after 24 hours

R-knee ↗

Fig. 1a/ after 1 hour

R-shoulder ↘

Fig. 1c/ after 72 hours

Fig. 1

## TECHNICAL FIELD

The present invention relates to a substance for specifically detecting an amyloid deposit and/or an amyloid deposition site. More specifically, the present invention relates to a substance for detecting amyloid deposition capable of accurately detecting an amyloid deposit and/or an amyloid deposition site caused by amyloidosis in such a manner as not to have a significant adverse effect on a living body.

## BACKGROUND ART

Amyloidosis is a disease in which an amyloid producing protein or a partial fragment of the amyloid producing protein deposits in a living body locally or systemically to form amyloid; a life-threatening condition, if symptoms are allowed to develop.

As reported by, for example, Isobe (Table 1); in recent years it has been made clear that amyloidosis can be categorized into several types (Nippon Clinical Medicine vol. 50, Hemocatharsis Therapy (second volume of two-volume set) pp. 394-399 (1992)).

| Range | Amyloid fibril protein | Clinical disease type | Biochemical characteristics of fibril protein | Precursor protein |
|---|---|---|---|---|
| Systemic | AL | Primary amyloidosis, part of myeloma, part of macroglobulin | Ig-L-chain($\kappa \cdot \lambda$-chain) or fragment, MW4-25K amyloid L-chain protein | Ig-L-chain($\kappa \cdot \lambda$-chain) |
| Systemic | AA | Secondary amyloidosis, reactive amyloidosis, tuberculosis, RA, lues, leprosy, pulmonary pyopoiesis, part of Hodgkin's disease etc., familial Mediterranean fever | Amino acid sequence basically common to that of an amyloid protein in an animal. Inconsistent with MW5-9 to 13K. AA Gly33, AA Trp33 etc. amyloid A protein | SAA, HDL$_2$ |
| | AF | Familial polyneuropathy, dysesthesia, autonomic disorder, hypotension, orthostatic hypotension | Different group transthyretin (old name: prealbumin) MW8 to 14K. amyloid protein, 14 kinds of variations such as Val$^{30}$ Met$^{30}$→ | Different group transthyretin |
| Systemic | AH | Amyloid complicated with prolonged hemodialysis, carpal tunnel syndrome, osteoarthropathy | $\beta_2$ microglobulin | $\beta_2$ microglobulin |
| Local | AE | medullary carcinoma of thyroid, Langerhans islet $\beta$-cell adenoma, pituitary adenoma | Fragment of prohormone related protein such as calcitonin, insulin, and prolactin | procarcitonin |
| | AHCH | Hereditary cerebral hemorrhage(island) | Different group C (Another name: $\gamma$-trace) fragment, MW11 to 15K | Different group cystathin C ($\gamma$-trace) |
| | ASb | Cerebrovascular amyloid, Alzheimer's disease, adult Down's syndrome | $\beta$-protein MW4.2K | ? |
| | ASC | senile (hereditary) (cardiovascular) amyloid | Different group transthyretin (old name: prealbumin) MW14K | Different group transthyretin |

(Nippon Clinical Medicine vol. 50. Hemocatharsis Therapy (second volume of two-volume set) pp. 394-399(1992)

**Table 1　Classification of amyloidosis and amyloid fibril protein**

For a long period of time, local or systemic amyloid deposition in a living body was detected by dissecting the living body. Amyloidosis was diagnosed based on the result of this detection.

However, it was known that depending upon the type of amyloidosis, appropriate treatments could enable the condition to be delayed, terminated, or improved; thus, the importance of an early diagnosis of amyloidosis was realized.

4

During the middle of this century, local or systemic amyloid deposits in a living body came to be detected through biopsy.

The detection of amyloid by biopsy can be conducted while a patient body is still living, thus attaining an early diagnosis of amyloidosis. However, the detection of amyloid by the biopsy procedure involves physical pain and tissue damage; therefore, a non-invasive detection method has been demanded.

In order to detect local or systemic amyloid deposition in a living body in a non-invasive manner, the detection method using a radioactive isotope has been attempted.

The detection method using a radioactive isotope utilizes the property of an amyloid producing protein which accumulates to form an amyloid deposition site. According to this method, an amyloid producing protein is allowed to be bound by a radioactive isotope for the purpose of labelling. The labelled amyloid producing protein is then intravenously administered to a living body. The protein thus administered is photographed from outside of the living body by using a scintillation camera or the like. In the event amyloid deposits exist in the patient, an amyloid deposition site in the living body where the radioactively labelled amyloid producing protein is deposited is obtained as an image, whereby the amyloid deposition site is detected. Thus, this method enables amyloidosis to be diagnosed in a non-invasive manner.

As the radioactive isotope used in the detection method utilizing a radioactive isotope: the radioactive isotopes selected to be used are those which can be easily measured from outside of a living body; allow as much diagnostic information to be obtained as possible; and have characteristics which allow for a small as possible dosage at radiation to be used, for example, those radioactive isotopes which do not radiate $\alpha$, $\beta^-$-rays (1), have a short physical half-life (2), and radiate a single $\gamma$-ray at 100 to 200 keV suitable for photo shooting by a scintillation camera or a single photon emission computed tomography (SPECT) apparatus (3), etc. Hitherto, radioactive isotopes such as iodine-123, iodine-131, and technetium-99m have been used for the diagnosis of amyloidosis. The advantages and disadvantages, kind, effective dosage amount, etc. of these radioactive isotopes are considered below.

Iodine-123 has a half-life as short as 13.3 hours, decays by electron capture (EC), and has a relatively small $\gamma$-ray energy of 159 keV; thus, it has slight biologically adverse effects. The use of iodine-123 is increasing with the advancement of a recent production technology. P.N. Hawkins et al. reported the diagnosis of primary and secondary amyloidosis using a serum amyloid P component labelled with iodine-123 (Lancet, 1, 1413-1418 (1988)). However, according to this method, a protein is labelled with iodine mainly by directly labelling a tyrosine residue; hence, it cannot be avoided that deiodinase present in a living body causes liberation of radioactive iodine from the protein. Consequently, the liberated iodine is known to accumulate in the thyroid gland. This causes concern about the adverse accumulative effect of the iodine on a living body. Furthermore, in the case where it takes several days to detect a deposition site, the required amount of iodine increases.

Iodine-131 is a radioactive isotope which has been the most widely used for labeling an antibody. This radioactive isotope has a relatively long half-life of 8.04 days, and even in the case where it takes several days to detect a deposition site, this isotope is more useful than the above-mentioned iodine-123 or technetium-99m described later. In fact, J. Floege et al. reported the diagnosis of dialysis related amyloidosis using $\beta$2-microglobulin labelled with iodine-131 (Kidney International, 38, 1169-1176 (1990)). However, besides its relatively long half-life of 8.04 days, iodine-131 has a $\gamma$-ray energy as large as 364 keV and radiates $\beta^-$-ray as well. Furthermore, iodine-131 requires the blocking of the thyroid gland in the same manner as in iodine-123. Accordingly, there is some doubt about the wide use of iodine-131 in the clinical field.

Technetium-99m has outstanding radioactive characteristics in terms of a clinical diagnosis: It has a very short half-life of 6.02 hours, decays by isomer transfer (IT), and has a $\gamma$-ray energy as small as 141 keV. In addition to this, Technetium-99m is easy to produce, and it is possible to design several functional labeling materials. These facts alone would allow Technetium-99m to be widely used; however, because of its very short half-life, a large amount of radioactive isotope is required in the case where the accumulation of amyloid to a deposition site is made clear only after the lapse of several days, for example, as in dialysis related amyloidosis. Thus, it is advantageous to label a protein with Technetium-99m.

As described above, the kind and amount of a radioactive isotope need to be determined by considering the effect of the radioactive isotope on a living body, the intensity of radiation needed for detection, the half-life thereof, etc. There are also problems such as the liberation of a labeling radioactive isotope and the accumulation thereof to a living body, as well as some difficulty in directly labeling an amyloid producing protein. Furthermore, operations for coping with these problems are complicated.

## DISCLOSURE OF THE INVENTION

The inventors of the present invention earnestly studied substances which enable the detection of amyloid deposition and/or a deposition site but which are less damaging to a living body while being capable of bringing about clear results. As a result, they found that the amyloid producing protein labelled with a radioactive isotope through a metal chelating substance is remarkably useful for the detection of amyloid deposition and a deposition site; thus achieving the present invention.

More specifically, according to the present invention, the above-mentioned conventional problems are solved, and a substance for detecting amyloid deposition which contains an amyloid producing protein bound by a metal chelating substance is provided.

According to the present invention, the above-mentioned substance for detecting amyloid deposition labelled with a radioactive isotope through a metal chelating substance is provided.

Furthermore, according to the present invention, a method for producing a substance for detecting amyloid deposition, including the step of allowing a metal chelating substance to be bound to an amyloid producing protein, followed by adding a radioactive isotope thereto is provided.

Still furthermore, according to the present invention, a method for detecting amyloid deposition or an amyloid deposition site, including the steps of: intravenously administering a substance for detecting amyloid deposition bound by (labelled with) a radioactive isotope into a living body, and detecting the substance for detecting amyloid deposition by a scintillation camera is provided.

Also, according to the present invention, the use of a substance containing an amyloid producing protein bound by a metal chelating substance for detecting an amyloid deposit in a living body or an amyloid deposition site is provided.

Also, the present invention includes the use of an amyloid producing protein and a metal chelating substance for producing a substance for detecting amyloid deposition.

Thus, according to the present invention, the objective of easily preparing a substance for detecting amyloid deposition useful for detecting amyloid deposition and/or a deposition site can be attained. In addition, the objective of detecting an amyloid deposit or an amyloid deposition site safely and accurately by detecting a labelled substance for detecting amyloid deposition with a scintillation camera can be attained.

## BRIEF DESCRIPTION OF THE DRAWINGS

Figure **1** shows images of amyloid deposition and deposition sites, obtained by intravenously injecting a substance for detecting amyloid deposition labelled with indium-111 into a living body, followed by photographing with a scintillation camera.

Figure **2** shows images of amyloid deposition and deposition sites obtained by intravenously injecting a substance for detecting amyloid deposition labelled with indium-131 into a living body, followed by photographing with a scintillation camera for comparison with indium-111.

## BEST MODE FOR CARRYING OUT THE INVENTION

The substance for detecting amyloid deposition of the present invention containing an amyloid producing protein bound by a metal chelating substance is obtained by allowing an amino group liberated from the amyloid producing protein to react with a metal chelating substance under the condition that the amino group can react with the metal chelating substance.

As the amyloid producing protein which can be used in the present invention, any of $\beta$2-microglobulin, serum amyloid P component, and an L-chain of immunoglobulin can be used as long as it can become a deposited protein characteristic of amyloidosis. These proteins can be prepared by general methods for purifying proteins. Examples of the methods include purification such as gel chromatography and ion exchange chromatography, dialysis, and lyophilization. Purification can be conducted by combining or repeating these methods.

The metal chelating substance which can be used in the present invention is required to have a metal chelating site capable of binding to a radioactive isotope. Examples of the metal chelating site capable of binding to a radioactive isotope include an ethylenediaminetetraacetic acid (EDTA) site and a diethylenetriaminepentaacetic acid (DTPA) site. Furthermore, the metal chelating substance used in the present invention is required to have at least one functional group capable of reacting with a protein to bind thereto.

6

Specific examples of the metal chelating substance satisfying the above-mentioned conditions include EDTA derivatives such as isothiocyanobenzyl-EDTA, aminobenzyl-EDTA, bromoacetamidobenzyl-EDTA, maleimidepropylamidobenzyl-EDTA, and maleimidepentylamidobenzyl-EDTA; and DTPA derivatives such as DTPA anhydride, isothiocyanobenzyl-DTPA, aminobenzyl-DTPA, bromoacetamidobenzyl-DTPA, maleimidepropylamidobenzyl-DTPA, and maleimidepentylamidobenzyl-DTPA. It is noted that the metal chelating substance is not limited to these examples.

According to the present invention, the amyloid producing protein can be bound by the metal chelating substance, for example, by adding the above-mentioned metal chelating substance to a protein solution in a buffer of pH 7-10, preferably pH 8-9, and stirring the mixture at, for example, 25°C to 37°C overnight under physiological conditions. This reaction is conducted considering the number of molecules of the metal chelating substance binding to one molecule of the protein. For example, if one molecule of the amyloid producing protein is bound by only 0.1 or less of metal chelating substance, inconvenience is caused in that the radioactivity is insufficient for monitoring from outside of a living body; thereby resulting in another inconvenience in that an excess of protein is required to be added in order to obtain sufficient radioactivity. When the metal chelating substance is allowed to be bound to all of the sites of the amyloid producing protein capable of being modified, the specificity of the amyloid producing protein disappears due to the change in its stereoscopic structure and the possibility of generating immune response is raised by excess modification of the protein. Thus, it is required to determine the number of molecules of the metal chelating substance bound to one molecule of the protein.

Furthermore, according to the present invention, the substance for detecting amyloid deposition containing an amyloid producing protein bound by a metal chelating substance is labelled with a radioactive isotope through the metal chelating substance.

As the radioactive isotope which can be used in the present invention, metal elements capable of forming a complex with the metal chelating substance are selected. Examples of the metal elements include indium-111 and technetium-99m. These elements have remarkable advantages that they can strongly bind to the metal chelating substance under the physiological conditions and do not require steps for protecting specified sites such as blocking of the thyroid gland.

The indium-111 has two relatively large $\gamma$-ray energies, i.e., 172 keV and 247 keV; however, its half-life is 2.81 days. Even considering that the accumulation of amyloid to a deposition site is not made clear until several days elapse in the case of the above-mentioned dialysis related amyloidosis, this half-life is appropriate. In addition, indium-111 has advantages in that since it decays by EC and radiates only $\gamma$-ray, the dosage of radiation in a living body is small, resulting in less adverse effect on the living body as compared with iodine-131.

The metal chelating substance bound to the amyloid producing protein is bound by a radioactive isotope by adding the radioactive isotope to a solution of the substance for detecting the amyloid deposition and stirring the mixture for an appropriate period of time. For example, a solution containing the above-mentioned radioactive isotope is added to the amyloid producing protein bound by the metal chelating substance and purified by gel chromatography. The mixture thus obtained is stirred for several hours and a free radioactive isotope is removed from the mixture by gel chromatography. This procedure can be conducted aseptically, and the resulting substance can be used as a sample for intravenous injection.

The substance for detecting amyloid deposition of the present invention is appropriate for clinical application for the following reasons: The amyloid producing protein previously allowed to be bound by the metal chelating material can be stored for a long period of time in this form. Since the amyloid producing protein previously allowed to be bound by the metal chelating material is not bound by radioactive isotopes, it can be easily stored. In the case where the metal chelating material bound to the amyloid producing protein is required to be labelled, labelling can be easily conducted by the above-mentioned method, right before being used for detecting amyloid deposition and deposition sites.

Furthermore, the labelled substance for detecting amyloid deposition can be prepared for intravenous injection as described above. Thus, amyloid deposition and/or a deposition site can be detected by intravenously administering the labelled substance and detecting the substance accumulated at a deposition site of the amyloid producing protein by a scintillation camera.

The substance of the present invention for detecting amyloid deposition is easily prepared and allows a detection result to be obtained in a shorter period of time compared with conventional methods. Therefore, the substance shortens the time burden on patients and is very notable in terms of safety. Furthermore, compared with images obtained by imaging with iodine-131, the radioactivity of a radioactive isotope used in the present invention enables a sharp image of an amyloid deposition site to be obtained. Therefore, the spread and range of a lesion can be accurately evaluated.

As described above, according to the present invention, the substance for detecting amyloid deposition which is very remarkable in terms of usefulness and safety in the detection of amyloid deposition and/or a deposition site, can be provided.

Example

Hereinafter, the present invention will be described in relation to $\beta$2-microglobulin; which is an amyloid producing protein in amyloidosis, especially dialysis related amyloidosis. The present invention is not limited to dialysis related amyloidosis and can be applied to deposited proteins caused by other specific amyloidosis; such as serum amyloid P component and an L-chain of immunoglobulin.

(1. Purification of $\beta$2-microglobulin)

Urine of a patient suffering from renal insufficiency, who is negative to human immunodeficiency virus (HIV) and hepatitis virus was ultrafiltrated to concentrate a $\beta$2-microglobulin-rich fraction. This concentrate was fractionated by gel chromatography (Sephadex G-75, produced by Pharmacia), followed by being purified by ion exchange chromatography (DEAE-Sephacel, produced by Pharmacia). The $\beta$2-microglobulin-rich fraction was dialyzed against distilled water at 4°C and lyophilized. The resulting $\beta$2-microglobulin-rich fraction was applied to high performance liquid chromatography (HPLC) using a gel filtration column (TSK Gel 2000SW, produced by Tosoh Corporation) to collect a fraction which was further rich in $\beta$2-microglobulin. The fraction thus collected was further purified by ion exchange chromatography and then HPLC, whereby further purified $\beta$2-microglobulin was obtained.

(2. Binding between $\beta$2-microglobulin and isothiocyanobenzyl-EDTA (SCN-Bz-EDTA))

A saturated solution of trisodium phosphate was added to a solution of phosphate buffer (0.15 M, pH 8.0) containing 5 mg of $\beta$2-microglobulin to adjust the pH of the buffer to 8.5. Then, 27.5 $\mu$l of a phosphate buffer (0.15 M, pH 8.0) containing 0. 55 mg of SCN-Bz-EDTA was added to the mixture and stirred at 30°C overnight. This reaction mixture was subjected to gel chromatography (Sephadex G-25, produced by Pharmacia) to remove any unreacted metal chelating substance. The resulting solution was kept at about 4°C. The resulting purified product which had been subjected to gel chromatography had almost the same elution volume as that of unmodified $\beta$2-microglobulin.

The number of molecules of the chelating substance bound to one molecule of the protein was confirmed by measuring the decrease in amino groups present on the surface of protein. First, the $\beta$2-microglobulin solution before the reaction and the reaction mixture before being purified by gel chromatography were sampled. The number of molecules of the metal chelating substance bound to one molecule of the protein was calculated by measuring the number of decreased amino groups per molecule of the protein, by a method using trinitrobenzenesulfonic acid. The result is shown in Table 2.

Table 2

| Number of decreased amino groups when equivalent weight of SCN-Bz-EDTA is reacted with $\beta$2-microglobulin | | |
|---|---|---|
| No. | Equivalent weight (eq.) | Number of decreased amino groups per molecule of protein |
| 1 | 1 | 0.6 |
| 2 | 3 | 0.9 |
| 3 | 10 | 2.2 |

(3. Introduction of indium-111)

First, 0.004 M hydrochloric acid solution of $^{111}InCl_3$ (indium 111, 185 MBq, produced by Amersham Corp.) was added to about 5 mg of $\beta$2-microglobulin bound by the metal chelating substance purified by gel chromatography. The mixture was stirred for several hours. Then, the mixture was aseptically subjected to gel chromatography using Sephadex G-25 to remove free indium, whereby a sample for intravenous injection radiolabelled with a radioactivity of about 74 MBq was obtained.

(4. Example of application)

A sample for intravenous injection radiolabelled with a radioactivity of about 74 MBq obtained by adding indium-111 to β2-microglobulin bound by the metal chelating material under the condition of No. 2 in Table 2 right before the detection of amyloid, was intravenously injected to a patient K.N. suffering from dialysis related amyloidosis. As shown in Figure **1**, images of amyloid deposition and deposition sites were obtained by using a scintillation camera one hour after the injection.

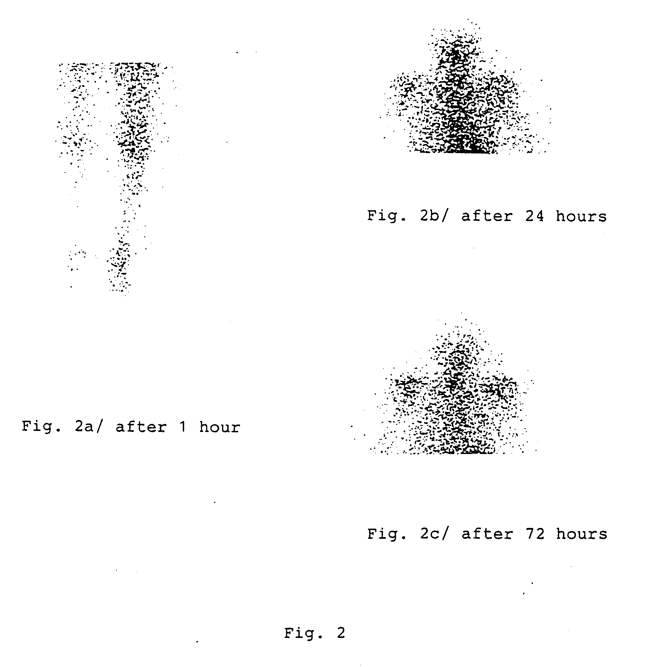

As a comparative example, the identical patient K.N. was imaged by using amyloid protein labelled with iodine-131. The result (in Figure **2**) was compared with the result shown in Figure **1**. Consequently, the following observation was obtained concerning indium-111: (1) A clear image is obtained; (2) Amyloid deposition and an amyloid deposition site can be detectable in a shorter period of time compared with the method using iodine-13 1; (3) indium-111 is hardly accumulated to a liver; and (4) No accumulation of indium-111 in the thyroid gland is found.

As is understood from the above results; indium-111, which is a radioactive isotope used in the present example, radiates only γ-ray so that a clear image of an amyloid deposition site can be obtained. On the other hand, iodine-131 radiates β⁻-ray in addition to γ-ray which makes the background of an image unclear because of its irregular reflection; therefore, it takes at least 2 days to obtain an image from which the accumulation of the amyloid producing protein can be detected. Thus, the image obtained from iodine-131 is unclear compared with the image obtained by using indium-111.

As described above, the present invention has been described illustrating indium-111. Technetium-99m can also be labelled in the same way as in indium-111. Although technetium-99m is not appropriate in the case where it takes several days to detect deposition sites as described above, this radioactive isotope is useful according to the present invention which enables the result of the detection to be obtained in a shorter period of time, compared with the conventional method.

**Claims**

1. A substance for detecting amyloid deposition comprising an amyloid producing protein bound by a metal chelating substance.

2. A substance for detecting amyloid deposition according to claim 1, labelled with a radioactive isotope through the metal chelating substance.

3. A substance for detecting amyloid deposition according to claim 2, wherein the radioactive isotope is indium-111.

4. A substance for detecting amyloid deposition according to claim 2, wherein the radioactive isotope is technetium-99m.

5. A substance for detecting amyloid deposition according to claim 1, wherein the amyloid producing protein is β2-microglobulin or a dimer thereof.

6. A substance for detecting amyloid deposition according to claim 1, wherein the amyloid producing protein is a partial peptide of β2-microglobulin.

7. A substance for detecting amyloid deposition according to claim 1, wherein the metal chelating substance has an ethylenediaminetetraacetic acid site.

8. A substance for detecting amyloid deposition according to claim 1, wherein the metal chelating substance has a diethylenetriaminepentaacetic acid site.

9. A substance for detecting amyloid deposition according to claim 1, wherein the metal chelating substance is introduced to such a degree as not to cause disruption of a stereoscopic structure of the amyloid producing protein.

**10.** A method for producing the substance for detecting amyloid deposition of claim 2, comprising the step of:

allowing the metal chelating substance to be bound to the amyloid producing protein, followed by adding the radioactive isotope thereto.

**11.** A method for detecting an amyloid deposit or an amyloid deposition site, comprising the steps of:

administering the substance for detecting amyloid deposition of claim 2 to a living body through an intravenous injection; and

detecting the substance for detecting amyloid deposition by a scintillation camera.

**12.** A use of a substance for detecting amyloid deposition comprising an amyloid producing protein bound by a metal chelating substance, for detecting an amyloid deposit or an amyloid deposition site in a living body .

**13.** The use according to claim 12, wherein the substance for detecting amyloid deposition is labelled with an radioactive isotope through the metal chelating substance.

**14.** The use according to claim 13, wherein the radioactive isotope is indium-111.

**15.** The use according to claim 13, wherein the radioactive isotope is technetium-99m.

**16.** The use according to claim 12, wherein the amyloid producing protein is $\beta$2-microglobulin or a dimer thereof.

**17.** The use according to claim 12, wherein the amyloid producing protein is a partial peptide of $\beta$2-microglobulin.

**18.** The use according to claim 12, wherein the metal chelating substance has an ethylenediaminetetraacetic acid site.

**19.** The use according to claim 12, wherein the metal chelating substance has a diethylenetriaminepentaacetic acid site.

**20.** The use according to claim 12, wherein the metal chelating substance is introduced to such a degree as not to cause disruption of a stereoscopic structure of the amyloid producing protein.

**21.** A use of an amyloid producing protein and a metal chelating substance for producing a substance for detecting amyloid deposition.

R-knee ↗

Fig. 1a/ after 1 hour

R-shoulder ↘

Fig. 1b/ after 24 hours

R-shoulder ↘

Fig. 1c/ after 72 hours

Fig. 1

Fig. 2b/ after 24 hours

Fig. 2a/ after 1 hour

Fig. 2c/ after 72 hours

Fig. 2

| International application No. |
| --- |
| PCT/JP94/02003 |

**A. CLASSIFICATION OF SUBJECT MATTER**

Int. Cl$^6$  A61K51/02, A61K49/00

According to International Patent Classification (IPC) or to both national classification and IPC

**B. FIELDS SEARCHED**

Minimum documentation searched (classification system followed by classification symbols)

Int. Cl$^6$  A61K51/02, A61K49/00

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
| --- | --- | --- |
| Y1 | JP, A, 3-502807 (Drocnoc Ltd.), June 27, 1991 (27. 06. 91) & GB, A, 2223578 & WO, A, 90/06777 | 1 - 10 |
| Y2 | JP, A, 56-164123 (Massachusetts Gener.), December 17, 1981 (17. 12, 81) & EP, A, 38546 & US, A, 4421735 | 1 - 10 |
| Y2 | JP, A, 63-267734 (Maeda H), November 4, 1988 (04. 11, 88)(Family: none) | 1 - 10 |
| Y2 | JP, A, 4-501503 (Centrocor. Inc.), March 19, 1992 (19. 03. 92) & WO, A, 89/11538 & EP, A, 418316 | 1 - 10 |
| A | WO, A, 93/04194 (Harvard College), March 4, 1993 (04. 03. 93) & EP, A, 599979 | 1 - 10 |
| A | Eur. J. Nucl. Med., vol. 16, No. 8-10, p.663-670 (1990), "Soft-tissue uptake of 99m | 1 - 10 |

| ☒ | Further documents are listed in the continuation of Box C. | ☐ | See patent family annex. |
| --- | --- | --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | | |
| "O" | document referring to an oral disclosure, use, exhibition or other means | "Y" | document of particular relevance; the claimed invention cannot be considered to involve an inventive step when the document is combined with one or more other such documents, such combination being obvious to a person skilled in the art |
| "P" | document published prior to the international filing date but later than the priority date claimed | "&" | document member of the same patent family |

| Date of the actual completion of the international search | Date of mailing of the international search report |
| --- | --- |
| April 3, 1995 (03. 04. 95) | April 25, 1995 (25. 04. 95) |

| Name and mailing address of the ISA/ | Authorized officer |
| --- | --- |
| Japanese Patent Office | |
| Facsimile No. | Telephone No. |

C (Continuation). DOCUMENTS CONSIDERED TO BE RELEVANT

| Category* | Citation of document, with indication, where appropriate, of the relevant passages | Relevant to claim No. |
|---|---|---|
| | Tc-diphosphonate and 99m Tc-pyrophosphate in amyloidosis" (Chemical Abstracts 113:128734) | 1 - 10 |
| A | J. Exp. Med., vol. 167, No. 3, p. 903-913 (1988), "Specific localization and imaging of amyloid deposits in vivo using iodine-123-labeled serum amyloid p component" (Chemical Abstracts 108:218309) | 1 - 10 |